# EUROPÄISCHE PATENTSCHRIFT

(11) **EP 3 202 435 B1**
(45) Veröffentlichungstag und Bekanntmachung des Hinweises auf die Patenterteilung: **13.10.2021**
(21) Anmeldenummer: 17154628.6
(22) Anmeldetag: 03.02.2017
(51) Int. Cl.: A61M 1/16

(54) **FILTERMODUL-VERPACKUNGS-EINHEIT**
FILTER MODULE PACKAGING UNIT
UNITÉ D'EMBALLAGE DE MODULE DE FILTRE

(30) Priorität: 05.02.2016 DE 102016102084
(43) Veröffentlichungstag der Anmeldung: 09.08.2017
(73) Patentinhaber: B. Braun Avitum AG, 34212 Melsungen (DE)
(72) Erfinder: DÖRING, Stefan, 01326 Dresden (DE)
(74) Vertreter: Winter, Brandl - Partnerschaft mbB

(56) Entgegenhaltungen:
- WO-A1-2015/147305
- WO-A1-2015/171876
- DE-A1-102007 036 734
- US-A1- 2005 063 859
- US-A1- 2014 124 397

## Beschreibung

Die Erfindung betrifft eine sterile Filtermodul-/Dialysatorverpackungseinheit, aufweisend ein in einer Primärverpackung steril verpacktes Filtermodul/einen Dialysator (Filterpatrone, Reinigungsfilter für Blutbehandlungsmaschinen, etc.).

Bei der Herstellung steriler Medizinprodukte, insbesondere Filtermodule/Dialysatoren, ist sicherzustellen, dass das Produkt bis zur Anwendung an einem Patienten oder Verwendung im Rahmen einer Behandlung steril bleibt. Dazu ist zu gewährleisten, dass entweder die Sterilbarriere an das Produkt gelegt wird oder dass die Verpackung eine Sterilbarriere gegenüber der Umgebung ausbildet, die unter Annahme realistischer Lagerbedingungen die auf dem Produkt angegebene Haltbarkeitsdauer (Shelflifetime) über unverletzt bleibt.

Filtermodule/Dialysatoren sind in ihrer äußeren Form ausschließlich im Hinblick auf fertigungs- und anwendungstechnische Erfordernisse ausgelegt. Es liegt auf der Hand, dass sich aus dieser Form besondere Anforderungen an die Filtermodul-/Dialysatorverpackung ableiten. Problematisch hinsichtlich der Verpackung sind insbesondere am Filtermodul/Dialysator standardisierte, abstehende und scharfkantige Konnektoren, Kanten am Filtermodul/Dialysator sowie an Schutzkappen (Protection Caps) des Filtermoduls/Dialysators.

Bekannte Verpackungen für Medizinprodukte, insbesondere für Filtermodule/Dialysatoren, bestehen primär aus einem Kunststoff- oder Aluminiumschlauch oder einem Siegelrandbeutel (als Primärverpackung) sowie aus einem Tray aus Kunststoff, Pappe oder Faserguss und ggf. einem Umkarton (als Sekundärverpackung). Insbesondere das Tray weist meist eine Form auf, die im Wesentlichen der Form des in der Primärverpackung verpackten Produkts entspricht, so dass eine Art Formschluss gegeben ist, mittels dem eine lagesichere Verpackung erstrebt wird.

US 2005/0063859 A1 zeigt eine Container-Verpackung für eine Blutbehandlungsvorrichtung mit Hohlfasern. Die Blutbehandlungsvorrichtung wird vor dem Sterilisationsprozess in den Container eingelegt, der einen Getter zur Sauerstoffabsorption aufweist, und dann verschlossen.

US 2014/0124397 A1 offenbart eine Vakuumverpackung für medizinisches Packgut. Das Packgut wird in einen offenen Bereich eines starren Containers eingelegt, der Container mit einer flexiblen Hülle umverpackt und vakuumiert. Der Container zeigt weiter eine Indikatorvorrichtung, die im vakuumierten Zustand der Verpackung eine vorbestimmte Stellung einnimmt und von einer Feder bei Vakuumverlust ihre Position verändert, sodass der Vakuumverlust von außen erkennbar ist.

WO 2015/147305 A1 zeigt eine Verpackung mit zwei Gettern, in der eine medizinische Spritze formschlüssig gelagert ist.

DE 10 2007 036 734 A1 offenbart ein Verfahren zur Gruppensterilisation von Gegenständen.

Einige Medizinprodukte, insbesondere Filtermodule/Dialysatoren, müssen ggf. unter sauerstofffreien Bedingungen sterilisiert werden. Das bedeutet, dass zum Zeitpunkt der Sterilisierung das Innere der Primärverpackung absolut sauerstofffrei sein muss. Dies wird in aller Regel durch Absorption des Sauerstoffs durch einen geeigneten Träger, einen sogenannten Getter, realisiert. Das Trägermaterial kann dabei beispielsweise Eisenpulver oder ein Polymer sein. Der Absorber kann als sogenanntes Sachet der Primärverpackung zugeführt werden oder in der Struktur des Verpackungsmaterials (Folie) enthalten sein.

Es ist ein beträchtlicher Nachteil, dass eine Bindung von molekularem Sauerstoff im abgeschlossenen System der Primärverpackung eine Volumenreduzierung bzw. einen Unterdruck (bei einer formunveränderlichen Umgebung) zur Folge hat. Bekannte Verpackungssysteme sind nicht formstabil, folglich reduziert sich ihr Volumen entsprechend der Sauerstoffbindung nach Verschließen der Verpackung in nicht kontrollierbarer Weise. Eine solche Volumenreduzierung der Primärverpackungen ermöglicht Relativbewegungen zwischen verpackten Filtermodulen/Dialysatoren untereinander innerhalb der Sekundärverpackung sowie zwischen verpackten Filtermodulen/Dialysatoren und der Sekundärverpackung, wobei diese Relativbewegungen wiederum zu Beschädigungen der Sterilbarriere führen können.

Bei bekannten Filtermodul-/Dialysatorverpackungen wird der vorstehend beschriebenen Problematik von Relativbewegungen infolge einer Volumenreduzierung bei Sterilisierung und eine daraus folgende mögliche Schädigung der Sterilbarriere durch Einsatz entsprechend dicker Folien und/oder einer sauerstoffreduzierten Atmosphäre während eines Verpackungsprozesses entgegengewirkt. Beide Vorgehensweisen bewirken in nachteiliger Weise hohe Material- bzw. Verfahrenskosten. Ein weiterer Nachteil ist, dass Volumenreduzierungen durch Vorsehen einer sauerstoffreduzierten Atmosphäre im Verpackungsprozess nicht vollständig ausgeschlossen werden können, da in einer derartigen Verpackung, insbesondere in einer Filtermodul-/Dialysatorverpackung 100%-ige Sauerstofffreiheit gewährleistet sein muss. In der Folge sind ein Einsatz eines Absorbers und daraus folgende Volumenreduzierungen unumgänglich. Dickere, mechanisch belastbarere Verpackungsmaterialien für die Primärverpackung erhöhen die Produktkosten und gewähren dennoch keine 100%-ige Sicherheit für die Unversehrtheit der Sterilbarriere.

Ausgehend von dem vorstehend beschriebenen Stand der Technik liegt der vorliegenden Erfindung die Aufgabe zugrunde, die zuvor angeführten Nachteile zu beseitigen, insbesondere eine sterile Filtermodul-/Dialysatorverpackung bereit zu stellen, mittels der Beschädigungen der Sterilbarriere infolge von Relativbewegungen zwischen Verpackungen, die durch unkontrollierte Volumenreduzierung möglich werden, minimiert oder vorzugsweise verhindert werden können. Die Verpackung selbst soll vorzugsweise gegenüber Volumenreduzierung unempfindlich sowie kostengünstig und formstabil sein.

Nach der Erfindung wird diese Aufgabe gelöst durch eine sterile Filtermodul-/ Dialysatorverpackungseinheit mit den Merkmalen des Anspruchs 1.

Durch die Reduktion an Sauerstoff im Aufnahmeraum kann das darin lagernde Filtermodul/der Dialysator abschließend einer Strahlen(Gamma)-Sterilisation unterzogen werden.

Die Verpackung eines Filtermoduls/Dialysators macht einen wesentlichen Bestandteil seiner Herstellungskosten aus. Durch die Erfindung kann in vorteilhafter Weise eine Senkung von Kosten erzielt werden, da Trays, wie sie bei Verpackungen nach dem Stand der Technik genutzt werden, nicht mehr erforderlich sind, um eine ausreichende Formstabilität der Verpackung trotz Volumenreduzierung durch Sauerstoffabsorption sicher zu stellen. Die Hartblisterverpackung nach der Erfindung ist besonders einfach und gut an die Form des verpackten Filtermoduls/Dialysators anzupassen (angepasst) oder anzunähern (angenähert). Relativbewegungen zwischen dem Filtermodul/Dialysator und der Verpackung können so auf ein Mindestmaß reduziert oder gar eliminiert werden. Durch genaue Formdefinition des Hartblisters, insbesondere dessen Formteils, ist eine automatisierte Verpackung zuverlässig und reproduzierbar möglich.

Im Sinne der Erfindung wird eine Verpackung mit einem Hartblister für Filtermodule/ Dialysatoren (oder auch anderen Medizinprodukte) genutzt. Dieser ist derart ausgebildet, dass trotz einer Volumenreduzierung oder eines im Aufnahmeraum entstehenden Unterdrucks infolge einer Bindung von Sauerstoff durch den Getter keine Relativbewegungen zwischen einzeln verpackten Filtermodulen/Dialysatoren möglich werden. Nach einer Ausführungsform kann das durch Definition eines Bereiches innerhalb der Verpackung erfolgen, an dem die Verpackung, insbesondere das Formteil sich infolge einer Volumenreduzierung ohne Beeinträchtigung der Gesamtstabilität und der grundsätzlichen Form der Verpackung verformen kann (Soll-Verformungsstelle/Soll-Verformungsbereich). Ohne Beeinträchtigung der grundsätzlichen Form der Verpackung bedeutet in diesem Sinne, dass bestimmte Außenbereiche der Primärverpackung, die dafür vorgesehen und angepasst sind, dass sich die Primärverpackung über diese bestimmten Außenbereiche zum Beispiel an anderen Primärverpackungen oder an einer Umverpackung (Sekundärverpackung) jeweils abstützt, sich nicht verformen. Ebenfalls im Wesentlichen unverformt bleiben (dadurch) Innenabschnitte der Primärverpackung, insbesondere des Formteils, über die oder an denen das Filtermodul/der Dialysator gehalten, gelagert oder abgestützt ist. D.h. jene bestimmten Außenbereiche des Formteils der Primärverpackung, die dazu angepasst sind, sich nicht (vernachlässigbar) zu verformen, bilden bevorzugt innenseitig auch jene Abschnitte, welche das eingelegte Filtermodul/Dialysator stützen.

Vorteilhafte Ausführungsformen der Erfindung sind in den Unteransprüchen beansprucht und werden nachfolgend näher erläutert.

Die erfindungsgemäße Verpackungseinheit ist dadurch gekennzeichnet, dass das Formteil im Wesentlichen formstabil ist (d.h. im normalen Betrieb/bei vorbestimmter Verwendung vernachlässigbar verformt wird). Es weist einen Druckausgleichsabschnitt auf, der als Volumenausgleichselement wirkt und gegenüber dem übrigen Formteil geschwächt ausgebildet ist. Geschwächt in diesem Sinne heißt, dass er mit geringerer Formstabilität als andere Bereiche des Formteils oder der (Primär)Verpackung ausgebildet ist. Auf diese Weise kann sich das Formteil infolge eines durch eine Bindung von Sauerstoff durch den Getter im Aufnahmeraum bewirkten Unterdrucks (im Wesentlichen) nur im Druckausgleichsabschnitt verformen. Im Übrigen kann es jedoch formstabil bleiben. Man kann auch sagen, dass die Primärverpackung ein Volumenausgleichselement aufweist, das bestimmt ist, nach einem hermetischen Verschließen der Primärverpackung auftretende Volumenänderungen, insbesondere Volumenänderungen aufgrund einer Bindung von Sauerstoff durch den Getter, (in den Soll-Verformungsbereichen) auszugleichen. Kernpunkte dieser Ausführungsform sind unter anderem die Aspekte der regionalen Schwächung zur Erreichung einer kontrollierten und definierten Volumenreduzierung bei Bindung von im Aufnahmeraum vorliegendem Sauerstoff ohne Schwächung und insbesondere unkontrollierte Formänderung der Gesamtverpackung vorzugsweise in den Bereichen, an denen sich die Primärverpackungen gegenseitig abstützen und weiter vorzugsweise in den Bereichen an denen das eingelegte Filtermodul/der Dialysator gestützt wird.

Bei einer weiteren Ausführungsform kann der Druckausgleichsabschnitt durch eine im unbelasteten Zustand sich nach außen wölbende Ausformung, insbesondere linsenförmige Ausformung, ausgebildet sein. Die Ausformung kann sich infolge eines im Aufnahmeraum herrschenden Unterdrucks aufgrund Bindung von Sauerstoff durch den Getter in eine nach innen wölbende Ausformung umformen. Im unbelasteten Zustand bedeutet in diesem Sinne, dass in der Verpackung kein Unterdruck herrscht.

Eine Ausführungsform der Erfindung ist dadurch gekennzeichnet, dass das Filtermodul/der Dialysator einen im Wesentlichen zylinderförmigen Mittenabschnitt aufweist, mit dem es/er an einem Lagerabschnitt des Formteils abgestützt und formschlüssig gehalten ist. Das Formteil kann beiderseits des Lagerabschnitts jeweils eine gegenüber der Außenkontur des Filtermoduls/Dialysators aufgeweitete Anschlussaufnahmestruktur ausbilden. In dieser können jeweils endseitig des Mittenabschnitts des Filtermoduls/Dialysators ausgebildete Filtermodul-/ Dialysatoranschlüsse aufgenommen sein. Vorzugsweise sind die Filtermodul-/ Dialysatoranschlüsse in der jeweiligen Anschlussaufnahmestruktur offen, also durch die Verpackung, insbesondere durch das Formteil oder das Deckelteil unversperrt. Sie sind demnach frei zugänglich und nicht durch die Primärverpackung abgedeckt, so dass eine strömungstechnische Verbindung des Inneren des Filtermoduls/Dialysators mit dem Aufnahmeraum, insbesondere mit dem Anschlussaufnahmevolumen, ausgebildet ist. Über diese strömungstechnische Verbindung kann im Inneren des Filtermoduls/Dialysators vorliegender Sauerstoff austreten und vom Getter gebunden werden. Eine Sauerstoffentfernung aus dem Filtermodul/Dialysator zum Zwecke einer Strahlen(Gamma)-Sterilisation ist auf diese Weise einfach und effektiv möglich.

Eine Ausführungsform ist dadurch gekennzeichnet, dass der Lagerabschnitt zumindest teilweise halbzylinderförmig ausgebildet ist. In einem solchen Lagerabschnitt ist ein zylinderförmiger Mittenabschnitt des Filtermoduls/Dialysators sowohl in radialer als auch in axialer Richtung geführt und gelagert aufnehmbar. Dabei können die Abmessungen von Filtermodul/Dialysator und Lagerabschnitt derart aufeinander abgestimmt sein, dass das Filtermodul/der Dialysator leicht geklemmt in dem Lagerabschnitt gehalten ist. Infolge der halbzylinderförmigen Geometrie des Lagerabschnitts ist das Filtermodul/der Dialysator durch einen Nutzer aber dennoch einfach aus der Verpackung zu entnehmen. Eine Klemmung kann allerdings auch durch die Protection Caps, die verschiedenen Konnektoren sowie jede andere Kontur am Filtermodul/Dialysator erreicht werden.

Eine erfindungsgemäße Filtermodul-/Dialysatorverpackungseinheit kann des Weiteren eine Sekundärverpackung aufweisen, in der eine Anzahl, vorzugsweise Mehrzahl von Primärverpackungen mit jeweils zumindest einem darin aufgenommen Filtermodul/Dialysator angeordnet sind, derart, dass die Primärverpackungen formschlüssig ineinander greifen. Ein besonders guter Formschluss zwischen den Primärverpackungen kann erzielt werden, indem diese, vorzugsweise das Formteil, insbesondere im Bereich des Anschlussaufnahmevolumens, in einem Querschnitt quer zur Längsrichtung des Filtermoduls/Dialysators eine im Wesentlichen P-förmige oder doppelt P-förmige Gestalt besitzt. Vorzugsweise ist die Verpackung derart konzipiert, dass das Filtermodul/der Dialysator im Kopf des P und ein Filtermodul-/ Dialysatoranschluss im Fuß des P aufgenommen ist. Bei dieser wie auch bei anderen Formen der Primärverpackung ist der Querschnitt der Primärverpackung quer zur Längsrichtung des Filtermoduls/Dialysators derart ausgebildet, dass zwei Primärverpackungen umgekehrt aufeinander angeordnet stabil ineinander greifen. Kernpunkt dieser bevorzugten Ausführungsform ist eine Verzahnung oder gegenseitige Abstützung mehrerer in einer Sekundärverpackung enthaltenen Primärverpackungen zur Einschränkung von Relativbewegungen zwischen den letztgenannten.

Vorzugsweise sind in dem Formteil Anlageabschnitte, insbesondere Anlageflächen, ausgebildet. Diese sind zum Bewirken einer lagestabilen Anordnung an einer benachbarten Primärverpackung eingerichtet und geeignet. Das Volumenausgleichselement ist vorzugsweise außerhalb dieser Anlageabschnitte angeordnet, derart, dass sich die Anlageabschnitte bei einer Verformung des Volumenelements zum Volumenausgleich nicht verformen. Es ist von besonderem Vorteil für eine lagestabile Anordnung, wenn gemäß einer Ausführungsform eine Mehrzahl von Primärverpackungen in Längsrichtung zueinander jeweils hälftig versetzt angeordnet ist, so dass jeweils eine Anschlussaufnahmestruktur zweier Primärverpackungen in den zwischen den beiden endseitigen Anschlussaufnahmestrukturen einer dritten Primärverpackung ausgebildeten Zwischenraum eingreifen und die drei Primärverpackungen in Längsrichtung zueinander fixieren.

Zusammenfassend kann man sagen, dass die Grundidee, auf der die Erfindung beruht, ist, einen Hartblister für eine sterile Verpackung eines Filtermoduls/Dialysators zu verwenden. Das Design des erfindungsgemäßen Hartblisters erfolgt vorzugsweise derart, dass die äußere Gestalt des Filtermoduls/Dialysators in der Primärverpackung abgebildet ist, ein Gasaustausch (Sauerstoffaustausch) zwischen Produkt und Absorber dennoch ungehindert möglich ist. Eine (Primär)Verpackung nach der Erfindung kann demnach zwei Kernpunkte umfassen:
- erstens eine definierte Schwächungszone, die die zwangsläufig auftretende Volumenreduzierung (aufgrund des Vorhandenseins des Absorbers) kompensiert und damit eine Formstabilität der (Primär)Verpackung zumindest an den entscheidenden Bereichen gewährleistet und
- zweitens eine äußere stabile Gestalt, die mehrere Einzel-/Primärverpackungen ineinandergreifen lässt, so dass Relativbewegungen zwischen gruppierten Einzel-/ Primärverpackungen ausbleiben.

Eine Volumenreduzierung der Verpackung ist auf einen örtlich definierten Bereich beschränkt. Dabei wird die Gesamtstabilität der Primärverpackung im Wesentlichen nicht (vernachlässigbar) eingeschränkt. Bei einer Ausführungsform der Erfindung ist eine stabile Stapelbarkeit ohne zusätzliche Zwischenlagen in der Sekundärverpackung gewährleistet. Dazu ist die äußere Form des Hartblisters so gestaltet, dass mehrere erfindungsgemäße Hartblister ineinandergreifen und sich gegenseitig stützen. Man kann sagen, dass mehrere Einzelverpackungen oder Primärverpackungen in einem Verbund so ineinander greifen, dass Relativbewegungen zwischen Einzelverpackungen, insbesondere Relativbewegungen in Richtung der Längsachse der Einzelverpackungen, nur eingeschränkt möglich oder gar unmöglich sind. Aus diesem Grund ermöglicht die Erfindung, auf bislang für die Verpackung von Filtermodulen/Dialysatoren erforderliche Zwischenlagen (Trays) in der Sekundärverpackung verzichten zu können.

Mit der Erfindung werden unter anderem die folgenden Vorteile erzielt:
- Einsparung von Zwischenlagen (Trays),
- Verbesserte Möglichkeit der Automatisierung (Handling), was insbesondere zu einer Senkung von Herstellkosten führt,
- Ermöglichung einer hohe Anzahl von Filtermodulen/Dialysatoren im Karton, was insbesondere zu einer Senkung von Logistikkosten führt,
- neue Designmöglichkeiten durch gerade "Kommunikationsflächen" zwischen einzelnen Primärverpackungen sowie einer Sekundärverpackung und
- Einschränkung von diversen Relativbewegungen, die in der Regel zu Verletzungen der Verpackung und damit der Sterilbarrieren führen.

Die Erfindung wird im Folgenden anhand beispielhafter, nicht einschränkender und in den angehängten Figuren gezeigter Ausführungsformen näher erläutert. Dabei zeigt:
Figur 1 eine schematische perspektivische Darstellung einer sterilen Filtermodul-/ Dialysatorverpackungseinheit bzw. eine Dialysator-Primärverpackung in einer ersten Ausführungsform,
Figur 2 eine Mehrzahl steriler Dialysator-Primärverpackung der Figur 1 gestapelt zum Umverpacken in eine Sekundärverpackung,
Figur 3 eine seitliche Ansicht einer zweiten Ausführungsform einer sterilen Dialysator-Primärverpackung,
Figur 4 die Dialysator-Primärverpackung der Figur 2 in geschlossener Umverpackung,
Figur 5 eine seitliche Ansicht zweier Dialysator-Primärverpackungen gemäß Figur 1 gestapelt zum Umverpacken.

In dem nachfolgend beschriebenem Ausführungsbeispiel wird von einem Dialysator und dessen Dialysator-Primärverpackung gesprochen. Es soll aber darauf hingewiesen werden, dass anstelle des genannten Dialysators jede andere Art eines Filtermoduls vorgesehen sein kann.

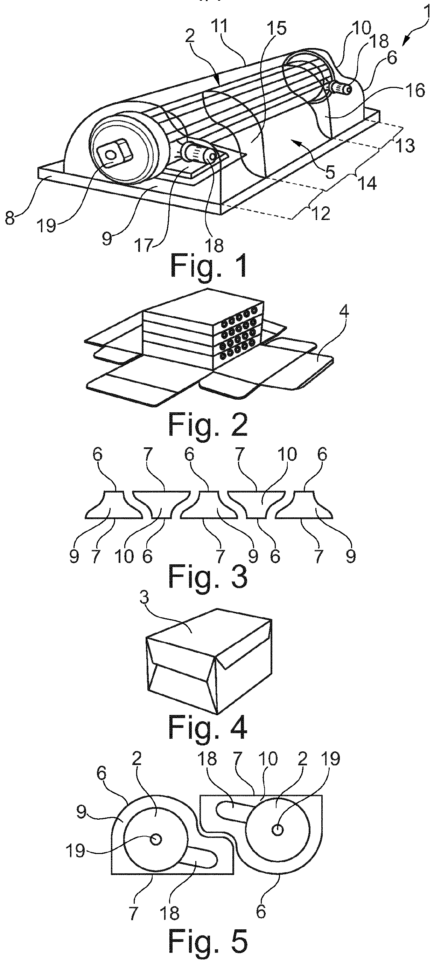

Die sterile Dialysator-Primärverpackung 1 (nachfolgend auch als Primärverpackung 1 bezeichnet) nach der Erfindung ist in Figur 1 mit einem steril darin verpackten Dialysator 2 gezeigt. Die Dialysator-Primärverpackung 1 wird zusammen mit weiteren Dialysator-Primärverpackungen 1 mit darin verpackten Dialysatoren mittels einer Sekundärverpackung oder Umverpackung 3 zu einer Verpackungseinheit kombiniert. Bei der Sekundärverpackung 3 handelt es sich zum Beispiel, wie in den Figuren 2 und 4 gezeigt ist, um einen Faltkarton 3, der aus einem Blank 4 hergestellt wird.

Die Primärverpackung 1 ist nach der Erfindung als Hart-Hart-Blisterverpackung mit einem einen Aufnahmeraum 5 für den Dialysator 2 ausbildenden Formteil 6 oder Unterteil 6 realisiert. Der Aufnahmeraum 5 ist nach bestimmungsgemäßer Anordnung des Dialysators 2 darin hermetisch einem am Formteil 6 angeordneten Deckelteil 7 verschlossen. Das Deckelteil 7 ist eine Kunststofffolie, die auf einem peripheren umlaufenden Rand 8 des Formteils 6 angeordnet und mit diesem hermetisch dicht verklebt ist.

Das Formteil 6 ist aus Kunststoff ausgebildet, beispielsweise durch Molding, und besitzt eine definierte Form mit zwei stabilen Seitenwänden 9, 10 und einer zwischen diesen angeordneten Wanne 11. Die Seitenwände 9, 10 und die Wanne 11 bilden den Aufnahmeraum 5 für den Dialysator 2 aus und sind an dessen offener Seite mit dem umlaufenden Rand 8 verbunden. Die jeweils an die entsprechende Seitenwand 9 bzw. 10 angrenzenden Randabschnitte 12, 13 der Wanne 11 sind ebenfalls mit entsprechender Stabilität ausgebildet. Unter "stabil" in diesem Sinne ist zu verstehen, dass sich die Seitenwände 9, 10 und die Wannenabschnitte 12, 13 bei einem sich im Aufnahmeraum 5 bildenden Unterdruck aufgrund einer Bindung von Sauerstoff im Wesentlichen nicht verformen. Zwischen den beiden seitlichen Wannenabschnitten 12, 13 ist ein mittlerer Wannenabschnitt 14 ausgebildet, der eine gegenüber den seitlichen Wannenabschnitten 12, 13 verminderte Formstabilität besitzt. Verminderte Formstabilität in diesem Sinne bedeutet, dass sich dieser Wannenabschnitt 14 bei einem sich im Aufnahmeraum 5 bildenden Unterdruck aufgrund einer Bindung von molekularem Sauerstoff zumindest teilweise aus einem ersten Formzustand in einen zweiten Formzustand verformt und einen Druckausgleich herbeiführt. Dabei bleiben die aus den Seitenwänden 9, 10 und den seitlichen Wannenabschnitten 12, 13 gebildeten Bereiche des Formteils 6 im Wesentlichen unverformt.

Bei der in Figur 1 gezeigten Ausführungsform des Formteils 6 ist der mittlere Wannenabschnitt 14 über Seitenwandabschnitte 15, 16 mit dem jeweiligen seitlichen Wannenabschnitt 12, 13 verbunden. Vorzugweise sind diese Seitenwandabschnitte 15, 16 stabil und die vorstehend angesprochene Verformung des Wannenabschnitts 14 erfolgt im Wesentlichen dazwischen. Die Seitenwandabschnitte 15, 16 bilden zusammen mit den seitlichen Wannenabschnitten 12, 13 Anlagestrukturen oder Anlageformen aus, an denen sich andere Formteile 6 mit ihrem mittleren Wannenabschnitt 14 bei Verpackung in der Umverpackung 3 lagestabil abstützen können. Dabei greift der mittlere Wannenabschnitt 14 einer ersten Primärverpackung 1 in den zwischen dem entsprechenden Seitenwandabschnitt 15 einer zweiten Primärverpackung 1 und dem entsprechenden Seitenwandabschnitt 16 einer dritten Primärverpackung 1 gebildeten Zwischenraum ein. Durch Anlage des mittleren Wannenabschnitts 11 der ersten Primärverpackung 1 an den seitlichen Wannenabschnitten 12, 13 der zweiten bzw. dritten Primärverpackungen 1 sind diese in radialer Richtung gelagert und abgestützt. Durch gegenseitige Anlage der Seitenwandabschnitte 15, 16 der Primärverpackungen 1 sind diese in axialer Richtung gelagert und abgestützt.

Um molekularen Sauerstoff, der nach dem Verschließen der Primärverpackung 1 in der Atmosphäre des Aufnahmeraums 5 vorliegt, zu Binden und für eine Sterilisation des verpackten Dialysators aus der Atmosphäre zu entfernen, ist ein Getter 17 in dem Aufnahmeraum 5 platziert.

Die Innenkontur des Aufnahmeraums 5 ist derart ausgebildet, dass der Dialysator 2 durch Formschluss gegenüber dem Formteil 6 gelagert ist. Die stabilen Seitenbereiche des Aufnahmeraums 5, gebildet durch die Seitenwand 9 und den seitlichen Wannenabschnitt 12 bzw. durch die Seitenwand 10 und den seitlichen Wannenabschnitt 13, bilden ein Volumen aus, das größer als das Volumen der darin aufgenommenen Abschnitte des Dialysators 2 ist, so dass dessen radiale Dialysatoranschlüsse 18 und axiale Dialysatoranschlüsse 19 nicht von dem Formteil 6 oder dem Deckelteil 7 verschlossen sind. Auf diese Weise ist das Innere des Dialysators 2 strömungstechnisch mit der Atmosphäre des Aufnahmeraums 5 verbunden, so dass auch im Inneren des Dialysators 2 vorliegender molekularer Sauerstoff mittels des Getters 17 gebunden werden kann.

Die Figuren 3 und 5 verdeutlichen, wie eine Mehrzahl von Primärverpackungen 1 in einer Art 69-Anordung zueinander lagestabil zum Verpacken in der Umverpackung 3 angeordnet werden. In Figur 5 ist deutlich die P-förmige Seitenkontur der Primärverpackungen 1 zu erkennen, wobei der Dialysatorkörper im Kopf des P und die Dialysatoranschlüsse 18, 19 im Fuß des P aufgenommen sind.

### Bezugszeichen

- 1: Dialysator-Primärverpackung oder Primärverpackung
- 2: Dialysator
- 3: Sekundärverpackung oder Umverpackung/Umkarton
- 4: Blank
- 5: Aufnahmeraum
- 6: Formteil
- 7: Deckelteil
- 8: umlaufender Rand
- 9: Seitenwand
- 10: Seitenwand
- 11: Wanne
- 12: seitlicher Wannenabschnitt
- 13: seitlicher Wannenabschnitt
- 14: mittlerer Wannenabschnitt
- 15: Seitenwandabschnitt
- 16: Seitenwandabschnitt
- 17: Getter
- 18: Dialysatoranschluss
- 19: Dialysatoranschluss

## Patentansprüche

1. Sterile Verpackungseinheit eines medizinischen Filtermoduls, aufweisend mindestens ein in einer Primärverpackung (1) steril verpacktes Filtermodul, wobei die Primärverpackung (1) als Blisterverpackung mit einem einen Aufnahmeraum (5) für das Filtermodul (2) aufweisenden Formteil (6) und einem am Formteil (6) angeordneten und den Aufnahmeraum (5) hermetisch verschließenden Deckelteil (7) ausgebildet ist, und
wobei die Primärverpackung (1) mindestens einen Getter (17) zur Bindung von in dem Aufnahmeraum (5) vorliegenden molekularen Sauerstoff aufweist, **dadurch kennzeichnet, dass**
das Filtermodul (2) durch Formschluss gegenüber dem Formteil (6) gelagert ist und
das Formteil (6) im Wesentlichen formstabil und ein Druckausgleichsabschnitt (14) des Formteils (6) gegenüber dem übrigen Formteil (6) geschwächt ausgebildet ist, so dass sich das Formteil (6) infolge eines durch eine Bindung von Sauerstoff durch den Getter (17) im Aufnahmeraum (5) bewirkten Unterdruck im Wesentlichen nur im Druckausgleichsabschnitt (14) verformt und Innenabschnitte der Primärverpackung (1), an denen das Filtermodul (2) gelagert ist, im Wesentlichen unverformt bleiben.

2. Sterile Verpackungseinheit eines medizinischen Filtermoduls nach Anspruch 1, **dadurch gekennzeichnet, dass** der Druckausgleichsabschnitt (14) durch eine im unbelasteten Zustand sich nach außen wölbende Ausformung ausgebildet ist, die sich infolge eines im Aufnahmeraum (5) herrschenden Unterdrucks aufgrund Bindung von Sauerstoff durch den Getter (17) in eine nach innen wölbende Ausformung umformt.

3. Sterile Verpackungseinheit eines medizinischen Filtermoduls nach einem der vorstehenden Ansprüche, **dadurch gekennzeichnet, dass** das Filtermodul (2) einen im Wesentlichen zylinderförmigen Mittenabschnitt aufweist, mit dem er an einem Lagerabschnitt des Formteils (6) abgestützt und formschlüssig gehalten ist.

4. Sterile Verpackungseinheit eines medizinischen Filtermoduls nach Anspruch 3, **dadurch gekennzeichnet, dass** das Formteil (6) beiderseits des Lagerabschnitts jeweils eine gegenüber der Außenkontur des Filtermoduls (2) aufgeweitete Anschlussaufnahmestruktur ausbildet, in der jeweils endseitig des Mittenabschnitts des Filtermoduls (2) ausgebildete Dialysatoranschlüsse (18, 19) aufgenommen sind.

5. Sterile Verpackungseinheit eines medizinischen Filtermoduls nach Anspruch 4, **dadurch gekennzeichnet, dass** die Filtermodulanschlüsse (18, 19) in der jeweiligen Anschlussaufnahmestruktur offen und strömungstechnisch mit dem Aufnahmeraum (5), insbesondere mit dem Anschlussaufnahmevolumen verbunden sind.

6. Sterile Verpackungseinheit eines medizinischen Filtermoduls nach einem der Ansprüche 3 bis 5, **dadurch gekennzeichnet, dass** der Lagerabschnitt zumindest teilweise halbzylinderförmig ausgebildet ist.

7. Sterile Verpackungseinheit eines medizinischen Filtermoduls nach einem der vorstehenden Ansprüche, des Weiteren aufweisend eine Sekundärverpackung (3), in der eine Mehrzahl von Primärverpackungen (1) mit jeweils zumindest einem darin aufgenommen Filtermodul (2) angeordnet sind, derart, dass die Primärverpackungen (1) formschlüssig ineinander greifen.

8. Sterile Verpackungseinheit eines medizinischen Filtermoduls nach Anspruch 7, **dadurch gekennzeichnet, dass** die Primärverpackung (1) in einem Querschnitt quer zur Längsrichtung des Filtermoduls (2) eine im Wesentlichen P-förmige oder doppelt P-förmige Gestalt besitzt, wobei das Filtermodul (2) im Kopf des P und ein Filtermodulanschluss (18, 19) im Fuß des P aufgenommen ist.

9. Sterile Verpackungseinheit eines medizinischen Filtermoduls nach Anspruch 8, **dadurch gekennzeichnet, dass** der Querschnitt der Primärverpackung (1) quer zur Längsrichtung des Filtermoduls (2) derart ausgebildet ist, dass zwei Primärverpackungen (1) umgekehrt aufeinander angeordnet stabil ineinander greifen.

10. Sterile Verpackungseinheit eines medizinischen Filtermoduls nach einem der vorstehenden Ansprüche, **dadurch gekennzeichnet, dass** in dem Formteil (6) Anlageabschnitte ausgebildet sind, die zum Bewirken einer lagestabilen Anordnung an einer benachbarten Primärverpackung (1) eingerichtet und geeignet sind und dass der Druckausgleichsabschnitt (14) außerhalb dieser Anlageabschnitte angeordnet ist.

11. Sterile Verpackungseinheit eines medizinischen Filtermoduls nach einem der vorstehenden Ansprüche, **dadurch gekennzeichnet, dass** eine Mehrzahl von Primärverpackungen (1) in Längsrichtung zueinander jeweils hälftig versetzt angeordnet sind, so dass jeweils eine Anschlussaufnahmestruktur zweier Primärverpackungen (1) in den zwischen den beiden endseitigen Anschlussaufnahmestrukturen einer dritten Primärverpackung (1) ausgebildeten Zwischenraum eingreifen und die drei Primärverpackungen (1) in Längsrichtung zueinander fixieren.

## Claims

1. A sterile packaging unit of a medical filter module comprising at least one filter module packed in a sterile primary package (1),
wherein the primary package (1) is a blister package comprising a molded part (6) including a receiving compartment (5) for the filter module (2) and a cover part (7) arranged on the molded part (6) and hermetically sealing the receiving compartment (5), and
wherein the primary package (1) includes at least one getter (17) for binding molecular oxygen present in the receiving compartment (5),
**characterized in that**
the filter module (2) is mounted by form closure vis-à-vis the molded part (6), and
the molded part (6) is configured to be dimensionally stable and a pressure compensation portion (14) of the molded part (6) is configured to be weakened as compared to the residual molded part (6) so that the molded part (6) will deform substantially only in the pressure compensation portion (14) due to vacuum caused in the receiving compartment (5) by the getter (17) binding oxygen, and inner portions of the primary package (1) on which the filter module (2) is mounted remain substantially undeformed.

2. The sterile packaging unit of a medical filter module according to claim 1, **characterized in that** the pressure compensation portion (14) is a configuration bulging outwards in the unloaded state, which will be formed into an inwards bulging configuration due to vacuum prevailing in the receiving compartment (5) by the getter (17) binding oxygen.

3. The sterile packaging unit of a medical filter module according to one of the preceding claims, **characterized in that** the filter module (2) includes a substantially cylindrical central portion by which it rests and is positively retained on a bearing portion of the molded part (6).

4. The sterile packaging unit of a medical filter module according to claim 3, **characterized in that** the molded part (6) on both sides of the bearing portion forms a port receiving structure expanded vis-à-vis the outer contour of the filter module (2), wherein dialyzer ports (18, 19) configured on each end side of the central portion of the filter module (2) are received in this port receiving structure.

5. The sterile packaging unit of a medical filter module according to claim 4, **characterized in that** the filter module ports (18, 19) are open in the respective port receiving structure and are in fluid communication with the receiving compartment (5), especially with the port receiving volume.

6. The sterile packaging unit of a medical filter module according to one of the claims 3 to 5, **characterized in that** the bearing portion at least partially has a semi-cylindrical shape.

7. The sterile packaging unit of a medical filter module according to one of the preceding claims, further comprising a secondary package (3) in which a plurality of primary packages (1) each including at least one filter module (2) received therein is arranged so that the primary packages (1) are in positive mesh.

8. The sterile packaging unit of a medical filter module according to claim 7, **characterized in that** the primary package (1) has a substantially P-shaped or double P-shaped design in a cross-section transversely to the longitudinal direction of the filter module (2), the filter module (2) being received in the head of the P and a filter module port (18, 19) being received in the foot of the P.

9. The sterile packaging unit of a medical filter module according to claim 8, **characterized in that** the cross-section of the primary package (1) is configured transversely to the longitudinal direction of the filter module (2) so that two primary packages (1) are in stable mesh when they are arranged inversely on top of each other.

10. The sterile packaging unit of a medical filter module according to one of the preceding claims, **characterized in that** in the molded part (6), contact portions are configured which are established and suited for bringing about a position-stable arrangement of an adjacent primary package (1), and **in that** the pressure compensation portion (14) is disposed outside of said contact portions.

11. The sterile packaging unit of a medical filter module according to one of the preceding claims, **characterized in that** a plurality of primary packages (1) is arranged to be offset relative to each other in the longitudinal direction by half of their longitudinal length so that a respective port receiving structure of two primary packages (1) will engage in the clearance formed between the two end-side port receiving structures of a third primary package (1) and will fix the three primary packages (1) relative to each other in the longitudinal direction.

## Revendications

1. Unité d'emballage stérile d'un module de filtre médical, présentant au moins un module de filtre emballé de manière stérile dans un emballage primaire (1), dans laquelle l'emballage primaire (1) est formé comme un emballage-coque avec une pièce moulée (6) présentant un espace de logement (5) pour le module de filtre (2) et une partie de couvercle (7) disposé sur la pièce moulée (6) et fermant hermétiquement l'espace de logement (5), et
dans laquelle l'emballage primaire (1) présente au moins un getter (17) destiné à fixer l'oxygène moléculaire présent dans l'espace de logement (5),
**caractérisée en ce que**
le module de filtre (2) est monté par complémentarité de forme par rapport à la pièce moulée (6) et
la pièce moulée (6) est formée sensiblement de manière à conserver sa forme et une portion d'équilibrage de pression (14) de la pièce moulée (6) est formée de manière réduite par rapport à la pièce moulée (6) restante, de sorte que la pièce moulée (6) en raison d'une pression négative induite par une fixation d'oxygène par le getter (17) dans l'espace de logement (5) est déformée sensiblement uniquement dans la portion d'équilibrage de pression (14) et des parties intérieures de l'emballage primaire (1), auxquelles est monté le module de filtre (2), restent sensiblement non déformées.

2. Unité d'emballage stérile d'un module de filtre médical selon la revendication 1, **caractérisée en ce que** la portion d'équilibrage de pression (14) est formée par une forme arquée vers l'extérieur dans un état non sollicité, qui se déforme en raison d'une pression négative dominante dans l'espace de logement (5) due à la fixation d'oxygène par le getter (17) dans une forme arquée vers l'intérieur.

3. Unité d'emballage stérile d'un module de filtre médical selon l'une quelconque des revendications précédentes, **caractérisée en ce que** le module de filtre (2) présente une portion centrale sensiblement cylindrique, par laquelle il s'appuie sur une portion d'appui de la pièce moulée (6) et est maintenu par complémentarité de forme.

4. Unité d'emballage stérile d'un module de filtre médical selon la revendication 3, **caractérisée en ce que** la pièce moulée (6) forme des deux côtés de la portion d'appui respectivement une structure de logement de borne élargie par rapport au contour extérieur du module de filtre (2), dans laquelle sont logées des bornes de dialyseur (18, 19) formées respectivement à l'extrémité de la portion centrale du module de filtre (2).

5. Unité d'emballage stérile d'un module de filtre médical selon la revendication 4, **caractérisée en ce que** les bornes de module de filtre (18, 19) sont ouvertes dans la structure de logement de borne respective et reliées fluidiquement avec l'espace de logement (5), en particulier avec le volume de logement de borne.

6. Unité d'emballage stérile d'un module de filtre médical selon l'une quelconque des revendications 3 à 5, **caractérisée en ce que** la portion d'appui est de forme au moins partiellement semi-cylindrique.

7. Unité d'emballage stérile d'un module de filtre médical selon l'une quelconque des revendications précédentes, présentant en outre un emballage secondaire (3), dans lequel une pluralité d'emballages primaires (1) sont disposés avec respectivement au moins un module de filtre (2) logé dans ceux-ci, de telle sorte que les emballages primaires (1) s'emboitent les uns dans les autres par complémentarité de forme.

8. Unité d'emballage stérile d'un module de filtre médical selon la revendication 7, **caractérisée en ce que** l'emballage primaire (1) possède dans une section transversale transversalement à la direction longitudinale du module de filtre (2) une configuration sensiblement en P ou en double P, dans laquelle le module de filtre (2) est logé dans la tête du P et une borne de module de filtre (18, 19) est logée dans le pied du P.

9. Unité d'emballage stérile d'un module de filtre médical selon la revendication 8, **caractérisée en ce que** la section transversale de l'emballage primaire (1) est formée transversalement à la direction longitudinale du module de filtre (2) de telle sorte que deux emballages primaires (1) disposés de manière à être retournés l'un sur l'autre s'emboitent manière stable l'un dans l'autre.

10. Unité d'emballage stérile d'un module de filtre médical selon l'une quelconque des revendications précédentes, **caractérisée en ce que** des portions d'appui sont formées dans la pièce moulée (6), qui pour obtenir un agencement de position stable sont installées et adaptées sur un emballage primaire (1) adjacent et **en ce que** la portion d'équilibrage de pression (14) est disposée à l'extérieur de cette portion d'appui.

11. Unité d'emballage stérile d'un module de filtre médical selon l'une quelconque des revendications précédentes, **caractérisée en ce qu'**une pluralité d'emballages primaires (1) dans la direction longitudinale les uns par rapport aux autres sont disposés de manière à être respectivement décalés de moitié, de sorte que respectivement une structure de logement de borne de deux emballages primaires (1) s'emboite dans l'espace intermédiaire formée entre les deux structures de logement de borne d'extrémités d'un troisième emballage primaire (1) et les trois emballages primaires (1) se fixent les uns par rapport aux autres dans la direction longitudinale.
